# FASCICULE DE BREVET EUROPEEN

(11) **EP 0 603 959 B1**
(45) Date de publication et mention de la délivrance du brevet: **11.09.1996**
(21) Numéro de dépôt: 93203572.8
(22) Date de dépôt: 17.12.1993
(51) Int. Cl.: A61F 2/06

(54) **Endoprothèse luminale et son procédé de préparation**
Endoluminale Prothese und ihre Herstellungsmethode
Endoluminal prosthesis and production method

(30) Priorité: 21.12.1992 BE 9201118
(43) Date de publication de la demande: 29.06.1994
(73) Titulaire: Dereume, Jean-Pierre, George, Emile, B-1050 Bruxelles (BE)
(72) Inventeur: Dereume, Jean-Pierre, George, Emile, B-1050 Bruxelles (BE)
(74) Mandataire: Claeys, Pierre

(56) Documents cités:
- EP-A- 0 461 791
- WO-A-92/06734
- DE-A- 3 918 736
- GB-A- 2 115 776
- US-A- 5 156 620

## Description

La présente invention est relative à une endoprothèse luminale, conforme au préambule de la revendication 1, ainsi qu'au procédé de préparation d'une telle endoprothèse.

On connaît déjà une endoprothèse luminale comportant un recouvrement expansible, appliqué sur la surface de paroi externe d'un tuteur tubulaire radialement expansible (voir US-A-4739762 et US-A-4776337). Dans ces deux brevets le procédé de fabrication n'est pas décrit et le recouvrement est formé d'un film mince élastique de polyuréthanne, de Teflon ou de matières biologiquement inertes. Ce film peut présenter des nervures en saillie radiale, comme moyens d'ancrage, ainsi que des ouvertures macroscopiques pour permettre le passage du sang entre le recouvrement et le vaisseau dans lequel l'endoprothèse est ancrée.

On connaît une prothèse luminale formée d'un corps tubulaire dont le diamètre peut être réduit par traction sur ses extrémités et qui est auto-expansible quand cette traction est supprimée (voir US-A-4.655.771 et US-A-4.954.126). Sur cette prothèse on peut éventuellement prévoir un recouvrement externe et/ou interne en une matière poreuse, élastique et biocompatible, par exemple en polyuréthanne. Aucun procédé de fabrication d'une telle prothèse recouverte n'est décrit dans le brevet, et rien n'est suggéré à propos de la manière d'obtenir la biocompatibilité escomptée et l'élasticité envisagée.

D'une manière correspondante, un recouvrement formé d'un film de polyuréthanne élastomère a été appliqué autour d'un tuteur métallique ayant des propriétés de mémoire de forme (A. BALKO et cons., Transfemoral Placement of Intraluminal Polyurethane Prosthesis for Abdominal Aortic Aneurysm, Journal of Surgical Research, 40, 305-309, 1986).

On a également décrit un dilatateur chirurgical formé d'un treillis auto-expansible revêtu ou noyé dans une matière élastique (GB-1205743).

La possibilité d'un recouvrement du tuteur d'une endoprothèse radialement expansible est également évoquée, mais non décrite, dans les US-A-5019090 et US-A-5092877.

Toutes ces endoprothèses luminales selon la technique antérieure présentent l'inconvénient de l'utilisation de matériaux totalement insuffisants en matière de biocompatibilité et de flexibilité.

Par ailleurs, on connaît déjà depuis longtemps des tuteurs tubulaires expansibles à utiliser seuls par exemple pour maintenir ouvertes les lumières de certains vaisseaux sanguins défaillants. On peut citer notamment les tuteurs à expanser par l'application d'une force extérieure, par exemple par le gonflement d'un ballonet situé à l'intérieur du tuteur (voir par exemple les brevets US-A-4733665, US-A-4739762, US-A-4776337, US-A-4800882, US-A-5019090). Comme autres tuteurs, on peut mentionner ceux qui sont auto-expansibles par élasticité propre (voir US-A-4580568; le tuteur mis sur le marché par PFIZER sous la dénomination de WALLSTENT®), ou par des propriétés de mémoire de forme (le tuteur mis sur le marché sous la dénomination de NITINOL).

On connaît également des prothèses semi-rigides introduites par voie endoluminale. Ces prothèses ont une forme de tubes pleins qui peuvent être accrochés aux vaisseaux à renforcer à l'aide de tuteurs d'extrémité tels que ceux mentionnés ci-dessus (voir EP-A-461791, US-A-4140126, US-A-4787899, US-A-5104399; J.C. PARODI et cons., Transfemoral Intraluminal Graft Implantation for Abdominal Aortic Aneurysms, Annals of Vascular Surgery, Vol. 5, n° 6, 1991).

Selon l'expérience actuelle, les tuteurs utilisés seuls ont montré qu'ils étaient la cause d'un développement anarchique des cellules dans les mailles du tuteur, avec pour effet une reformation rapide d'épaississements cellulaires à l'intérieur des vaisseaux à protéger, c'est-à-dire une hyperplasie fibreuse. D'autre part, les tubes introduits à l'heure actuelle comme endoprothèses sont utilisés sans tuteurs de support. Ils doivent donc offrir une certaine rigidité. Leur structure même les rend inappropriés du point de vue de la croissance cellulaire sur leurs parois. Pour ces mêmes raisons, les endoprothèses expansibles comprenant un tuteur revêtu d'un film expansible sont inappropriées pour la croissance normale des cellules. Elles continuent à représenter un corps étranger à l'intérieur du corps humain ou animal où elles sont implantées et les films utilisés excluent un envahissement cellulaire normal à l'intérieur même de leur structure. Ils présentent en outre une résistance à la dilatation en sens radial de l'endoprothèse telle qu'ils ont jusqu'à présent représenté un empêchement à leur utilisation sur des tuteurs, en particulier auto-expansibles.

Pour résoudre ces problèmes, on a prévu suivant l'invention une endoprothèse luminale telle qu'indiquée dans la revendication 1. Il est apparu de manière surprenante, qu'une matière fibreuse présentant des ouvertures entre fibres supérieures à 30µ, de préférence d'au moins 50µ, lorsque l'élément de tuteur présente son deuxième diamètre permettait non seulement un envahissement cellulaire, mais surtout une croissance normale de cellules avec une cohabitation parfaite entre cellules et fibres biologiquement compatibles.

Par le fait que la matière fibreuse est constituée de fibres orientées sous un angle inférieur à 50°, éventuellement inférieur à 30°, par rapport à une génératrice de l'élément de tuteur tubulaire, lorsque ce dernier présente son premier diamètre, et sous un angle supérieur, lorsque l'élément de tuteur présente son deuxième diamètre, la matière fibreuse est donc constituée de fibres qui vont se réarranger et se réorienter au fur et à mesure de l'augmentation ou de la diminution de diamètre du tuteur. Cela confère le grand avantage d'une "élasticité de structure" du recouvrement. Avantageusement, la matière fibreuse est de plus constituée de fibres elles-mêmes élastiques. On peut de préférence envisager une matière fibreuse constituée de fibres de polycarbonate, de préférence d'un uréthanne polycarbonaté. On peut par exemple utiliser de la fibre de polyuréthanne polycarbonaté mise sur le marché par la firme CORVITA Corp. sous la dénomination de CORETHANE®. Il est toutefois évident que d'autres substances peuvent être envisagées pour être mises en oeuvre dans la présente invention. Un tel recouvrement en matière fibreuse permet sans problème une extension de 2 à 4 fois son diamètre et même davantage. Dans cet état, on peut même considérer que le recouvrement a atteint une déformation permanente.

Suivant une forme de réalisation avantageuse de l'invention, la matière fibreuse est constituée de fibres d'un diamètre de 1 à 2µ disposées en plusieurs couches superposées. De préférence chacune des couches superposées susdites présente des fibres orientées de manière à croiser les fibres d'une couche voisine supérieure et celles d'une couche voisine inférieure.

On connaît des prothèses formées d'un tube en une matière fibreuse présentant une telle structure de couches superposées de fibres, dont les fibres d'une couche croisent celles des couches voisines, ainsi que le procédé de préparation de ces prothèses (voir US-A-4475972, US-A-5133742, J. LEIDNER et cons., A novel process for the manufacturing of porous grafts : Process description and product evaluation, Journal of Biomedical Materials Research, vol. 17, 229-247 (1983) ). Ces prothèses sont utilisées seules pour remplacer par exemple des fragments de vaisseaux défectueux. Ces prothèses sont peu dilatables en sens radial, non seulement elles n'en ont pas besoin, mais ce serait même un inconvénient en présence de la tension sanguine interne. Généralement les fibres sont superposées en environ 800 couches successives de fibres entrecroisées.

Par une adaptation de ce procédé de fabrication, il est apparu possible d'appliquer, directement sur un élément de tuteur tubulaire expansible, un recouvrement éminemment expansible lui aussi, bien que formé de couches superposées de fibres entrecroisées.

Suivant une forme perfectionnée de l'invention, l'élément de tuteur présente un recouvrement expansible appliqué sur la surface de paroi externe de l'élément de tuteur et/ou sur sa surface de paroi interne. Il est ainsi possible d'envisager des endoprothèses armées, dont l'élément de tuteur ne peut plus du tout venir en contact avec le tissu humain ou animal. Il en résulte une amélioration encore supérieure de la biocompatibilité.

Pour préparer une endoprothèse suivant l'invention, on prévoit un procédé tel qu'indiqué dans la revendication 15. Ce procédé est appliqué de préférence lors de l'utilisation d'un tuteur expansible par l'application d'une force extérieure, par exemple le gonflement d'un ballonet.

On peut aussi prévoir un procédé tel qu'indiqué dans la revendication 19. Ce procédé est appliqué de préférence lors de l'utilisation d'un tuteur auto-expansible.

D'autres détails et particularités de l'invention ressortiront des revendications, ainsi que de la description qui suit et qui est donnée à titre non limitatif, avec référence aux dessins annexés.

La figure 1 représente une forme de réalisation d'endoprothèse luminale suivant l'invention, à l'état d'introduction dans une voie d'un corps humain ou animal.

La figure 2 représente cette forme de réalisation après expansion radiale.

La figure 3 représente un dispositif pour la fabrication d'une endroprothèse luminale suivant l'invention.

Sur les figures, les éléments identiques ou analogues sont désignés par les mêmes références.

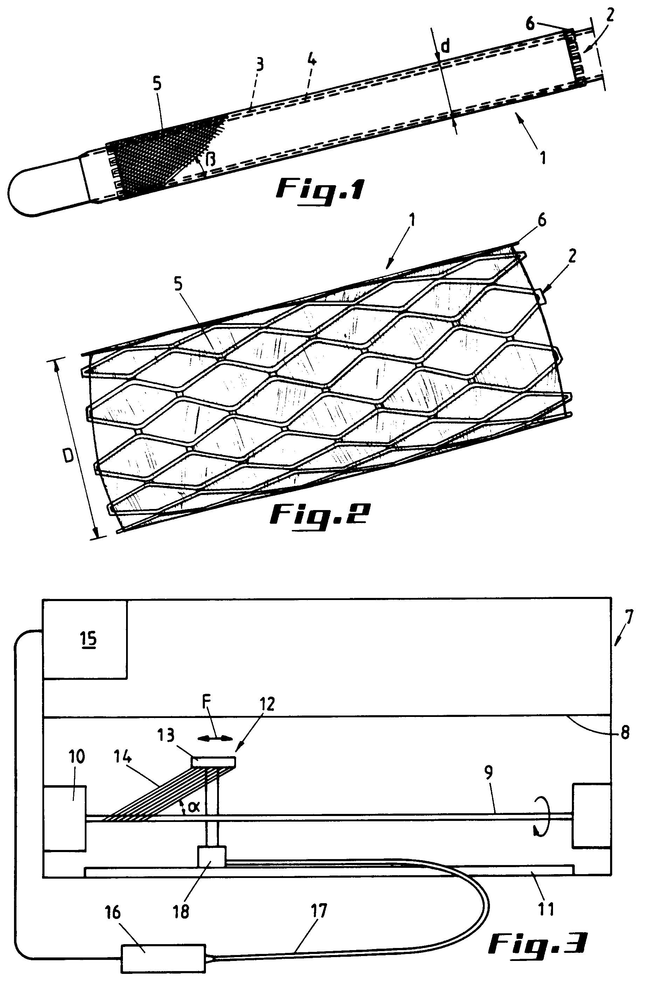

Sur les figures 1 et 2 on peut voir un exemple de réalisation d'endoprothèse luminale 1 suivant l'invention. Cette endoprothèse comprend un élément de tuteur tubulaire 2 expansible entre un premier diamètre d représenté sur la figure 1 et un deuxième diamètre D représenté sur la figure 2. Lorsque le tuteur présente son premier diamètre, il est en état d'être introduit dans une voie du corps humain ou animal, par exemple dans un vaisseau sanguin périphérique, puis d'être guidé jusqu'à l'emplacement recherché. Là le tuteur est dilaté de façon qu'il présente le diamètre D. Il faut noter que le deuxième diamètre D ne représente pas nécessairement le diamètre maximal d'expansion du tuteur, mais celui correspondant à son application contre les parois de la voie du corps humain ou animal à soutenir, à l'emplacement recherché. Le tuteur peut donc présenter encore un troisième diamètre encore supérieur, qui n'est pas représenté.

Le tuteur tubulaire, utilisé dans l'exemple de réalisation illustré, est un tuteur connu en soi, gonflable par un ballonet interne 3, connu et représenté en traits interrompus sur la figure 1. Le ballonet 3 est monté sur un cathéter 4. Il est évident que l'on pourrait aussi imaginer un tuteur tubulaire auto-expansible. Dans ce cas, le tuteur est entouré d'un manchon qui le retient dans cet état où il présente le diamètre d.

L'endoprothèse luminale suivant l'invention comporte également un recouvrement expansible 5, en une matière biologiquement inerte, appliqué sur la surface de paroi externe du tuteur. Ce recouvrement est en une matière fibreuse qui présente des ouvertures entre fibres supérieures à 30µ, de préférence d'au moins 50µ, lorsque le tuteur est dans l'état représenté sur la figure 2.

Ainsi qu'il ressort de la figure 1, les fibres sont, lorsque le diamètre du tuteur 2 est égal à d, orientées sous un angle β d'environ 30° par rapport à une génératrice du tuteur tubulaire. En fonction de ce qui est souhaitable comme expansion entre le premier diamètre d et le deuxième diamètre D, c'est-à-dire en fonction de la dilatation radiale que va subir l'endoprothèse, l'angle β entre les fibres et la génératrice du tuteur sera variable. Après dilatation radiale, le diamètre D sera de préférence compris entre 2 et 4 fois le diamètre initial d.

Lorsque le tuteur a atteint le diamètre D, les fibres se sont réorientées l'une par rapport à l'autre avec un agrandissement de l'angle entre elles-mêmes et ladite génératrice. Dans cette position, cet angle peut avoir atteint par exemple 45° ou davantage, par exemple 60°. Dans l'exemple illustré, non seulement le tuteur, mais aussi le recouvrement, ont atteint un état de déformation permanente.

La matière fibreuse elle-même sera avantageusement, bien que non nécessairement, élastique. On peut citer, comme exemple de fibres très appropriées, des fibres de polycarbonate mises sur le marché par la firme CORVITA Corp. sous la dénomination de CORETHANE®. L'élasticité des fibres favorise évidemment les propriétés d'expansion du recouvrement.

Dans l'exemple de réalisation illustré, le recouvrement est constitué de fibres de polycarbonate d'un diamètre de 1 à 2µ disposées en plusieurs couches, par exemple en 100 couches superposées ou davantage. Les fibres d'une couche sont disposées parallèlement l'une à l'autre, tandis que les fibres de la couche voisine supérieure et de la couche voisine inférieure sont disposées parallèlement entre elles mais de manière à croiser les fibres de la couche interposée. Les fibres peuvent être scellées entre elles à l'endroit des croisements. Cette disposition permet une expansion radiale pour autant que le nombre de couches superposées ne soit pas excessif et ne dépasse pas par exemple 500 couches. Elle permet également la formation recherchée d'ouvertures entre fibres à l'état dilaté du recouvrement.

Comme on peut le constater sur la figure 1, le tuteur 2 est dépourvu de recouvrement à ses deux zones d'extrémité 6. Lorsque le tuteur atteint son deuxième diamètre D, son expansion est limitée dans sa partie centrale par le recouvrement 5, mais pas dans les zones d'extrémité 6. Il en résulte que là les extrémités 6 du tuteur font radialement saillie vers l'extérieur et elles peuvent alors servir de moyens d'accrochage dans la paroi de la voie du corps à soutenir.

Il est compréhensible que la matière fibreuse du recouvrement peut non seulement être formée d'un non-tissé comme décrit, mais aussi de fibres tissées ou tricotées. On peut aussi prévoir un recouvrement sur la surface de paroi interne du tuteur ou un recouvrement sur les deux surfaces de paroi de celui-ci. Dans ce dernier cas le tuteur ne vient plus en contact avec le tissu du corps humain ou animal.

De telles endoprothèses suivant l'invention peuvent être introduites, comme déjà dit plus haut, dans un vaisseau sanguin périphérique et être acheminées jusqu'à une artère ou une veine dont la lumière est en voie d'obstruction, par exemple par un processus de cicratisation exagérée ou une croissance cellulaire anormale (hyperplasie fibreuse) ou au niveau de sténoses artérielles ou veineuses.

Elles permettent également de renforcer une paroi vasculaire affaiblie par un processus pathologique, par exemple une dissection pariétale, ou dilatée, comme dans le cas d'anévrismes.

Elles permettent aussi l'oblitération de communications artério-veineuses congénitales ou acquises. Elles peuvent être appliquées au niveau d'un shunt porto-cave intra-hépatique.

On peut aussi les mettre en oeuvre pour maintenir ouverte la lumière de conduits biologiques autres que sanguins, comme les voies digestives, biliaires, pancréatiques, urinaires, etc.. Elles permettent de limiter la croissance intraluminale de processus pathologiques, comme une fibrose ou un cancer.

L'avantage primordial et inattendu de ces endoprothèses suivant l'invention est qu'elles permettent un envahissement transpariétal du revêtement par du tissu vivant sans développement anarchique des cellules et sans tendance à une sténose ou à une resténose.

Des modes d'application d'endoprothèses suivant l'invention vont être décrits ci-dessous, à titre d'exemples non limitatifs.

### Exemple 1. : Implantation d'endoprothèse chez l'animal.

Une communication est créée chirurgicalement entre l'aorte et la veine cave inférieure de chiens.

La fermeture de cette communication est rendue possible par ouverture de l'artère fémorale du chien et introduction dans celle-ci d'un cathéter à ballon de dilatation sur lequel est monté une endoprothèse suivant l'invention, telle qu'illustrée sur la figure 1. L'ensemble, qui présente un diamètre de 3,4 mm, est amené sous contrôle angioscopique au site où la fistule aorto-cave a été créée. Le gonflement du ballon permet la dilatation de l'endoprothèse à un diamètre de 12 mm et l'incrustation des extrémités de l'endoprothèse dans la paroi aortique, en supprimant de ce fait la fistule aorto-cave. Le ballon est ensuite dégonflé et ôté du corps avec le cathéter.

Après un mois, les endoprothèses ont été explantées du site d'implantation. Un examen direct et une étude de coupes histologiques permettent d'affirmer une complète invasion de l'endoprothèse, et en particulier de son recouvrement, par du tissu vivant ainsi que la réhabitation de la face luminale par des cellules endothéliales. On constate en fait un dépôt luminal de collagène recouvert d'une couche de cellules néoendothéliales à la surface en contact avec le sang. Les barres du tuteur pourvu du recouvrement sont entourées de collagène et couvertes de néointima.

On n'a pas pu constater de signes histologiques de rejet ou d'hypperplasie fibreuse responsable des échecs cliniques observés lors de l'application d'endoprothèses selon l'état antérieur de la technique. On n'a observé aucune cellule géante à corps étranger, aucun macrophage.

### Exemple 2. : Implantation d'endoprothèse chez l'homme.

Fermeture d'une fistule artério-veineuse ilio-cave par implantation d'une endoprothèse dans l'artère iliaque porteuse de la fistule. L'endoprothèse comporte un tuteur métallique auto-expansible suivant l'invention de 12 mm de diamètre (Wallstent®) recouvert de 100 couches de fibres de polycarbonate (Corethane®) appliquées conformément à l'invention sur le tuteur métallique, lorsqu'il a son plus grand diamètre, avec un angle α de 80°.

Une traction manuelle sur l'endoprothèse permet de la réduire à son petit diamètre d et son introduction dans l'embout proximal d'un cathéter porteur, courant en soi, dont le diamètre interne est de 3,3 mm (produit de la firme Balt company). Ce cathéter porteur est préalablement introduit dans l'artère fémorale et son extrémité est positionnée dans l'artère iliaque porteuse de la fistule. L'utilisation d'un poussoir connu en soi, introduit dans l'embout proximal du cathéter porteur, permet de pousser l'endoprothèse jusqu'à son extrémité et la libération de l'endoprothèse dans l'artère iliaque ainsi que la fermeture immédiate de la fistule artério-veineuse à haut débit.

Un même dispositif a été utilisé avec succès pour traiter une dissection spontanée de l'artère iliaque droite avec développement d'un faux anévrisme, et une sténose serrée de l'artère iliaque gauche.

### Exemple 3. : Implantation d'endoprothèse chez l'homme.

Indication : volumineux anévrisme de l'aorte thoraco-abdominale fistulisé dans les voies aériennes et entraînant des hémoptysies. Matériel utilisé : endoprothèse suivant l'invention composée d'un tuteur métallique auto-expansible de 40 mm de diamètre et de 15 cm de longueur recouvert conformément à l'invention de 150 couches de fibres de polycarbonate appliquées sous un angle α de 80°. Le diamètre de l'endoprothèse est réduit par traction manuelle. Celle-ci est alors introduite dans la gaine d'un introducteur (Schneider company) d'un diamètre interne de 4,5 mm. L'endoprothèse est introduite dans l'extrémité distale de la gaine porteuse. L'endoprothèse est enfoncée dans la gaine porteuse jusqu'à 2 cm de l'extrémité distale, permettant de sertir dans l'extrémité distale un embout conique dont l'axe est percé d'un orifice. On aborde alors l'artère fémorale gauche du patient. Introduction d'un guide métallique sur lequel on a fixé une ligature chirurgicale à 15 cm de son extrémité. Le guide est alors amené et positionné dans l'aorte ascendante. Après introduction de l'extrémité proximale du guide à travers l'embout distal conique, l'introducteur est avancé jusqu'en position thoraco-abdominale grâce à un poussoir introduit dans l'extrémité proximale de l'introducteur. On libère ainsi l'endoprothèse en regard de l'anévrisme thoraco-abdominal, en permettant d'exclure la cavité anévrismale et sa fistule vers les bronches.

Un dispositif de ce genre a été utilisé avec succès notamment pour traiter un volumineux anévrisme développé au niveau de la crosse aortique et de l'aorte descendante.

Sur la figure 3, on a représenté un dispositif pour la mise en oeuvre d'un procédé de préparation d'endoprothèses suivant l'invention. Ce dispositif correspond sensiblement à celui décrit dans le US-A-4475972 et dans J. LEIDNER et cons., op. cit. Il comprend un bâti 7 supportant une enceinte fermée 8 à l'intérieur de laquelle est maintenue une température élevée permettant un scellage des fibres lorsqu'elles sont appliquées l'une sur l'autre. A l'intérieur de cette enceinte est supporté un mandrin 9 d'un diamètre juste inférieur au diamètre d du tuteur expansible. Ce mandrin 9 est entraîné en rotation autour de son axe longitudinal par un moteur rotatif 10. L'enceinte 8 contient en outre un rail 11 disposé parallèlement au mandrin 9 et sur lequel peut coulisser en va-et-vient suivant la direction F une filière désignée globalement par la référence 12. La filière 12 est entraînée sur le rail 11 par un moteur 18. La tête d'extrusion 13 de la filière est agencée juste au-dessus du mandrin 9 et comporte une multiplicité d'orifices par lesquels sortent les fils 14 de la matière extrudée. Celle-ci provient d'un réservoir 15, d'où elle est aspirée par une pompe 16 pour être refoulée sous pression dans un conduit flexible 17 qui débouche à la base de la filière 12. Celle-ci présente une chemise de refroidissement, non représentée en détail, qui maintient la matière à extruder, ici du polycarbonate, à une température de 35°C à la sortie de la filière.

Un tuteur présentant son premier diamètre est enfilé sur le mandrin 9 et est entraîné en rotation par ce dernier. On pourrait éventuellement envisager aussi un agencement du tuteur directement à la place du mandrin. La filière 12 est mise en marche en va-et-vient et les fils extrudés par la filière sortent de celle-ci avec un certain angle d'inclinaison α par rapport à l'axe du mandrin. Cet angle peut donc être réglé en fonction de la vitesse de la filière et de la vitesse de la rotation du moteur 10.

Lorsque la filière 12 arrive au bout du mandrin 9, à droite sur la figure 3, son sens de déplacement est inversé, d'une manière connue, par le moteur 18 et les fibres extrudées présentent également un angle α par rapport à l'axe du mandrin, mais dans le sens opposé à celui de la première couche. La deuxième couche de fibres présente donc des fibres disposées parallèlement mais croisant celles de la première couche.

Suivant un exemple de réalisation non limitatif, on a appliqué les paramètres de fabrication suivants :

| | |
|---|---|
| Température de la matière extrudée à la sortie de la tête d'extrusion | 35°C |
| Température à l'intérieur de l'enceinte 8 | 270°C |
| Humidité relative de l'air à l'intérieur de l'enceinte | 29% |
| Diamètre du mandrin | 3mm |
| Angle d'inclinaison des fibres par rapport à l'axe du mandrin | 30° |
| Vitesse du moteur linéaire 18 | 19,8 cm/s. |
| Flux des fibres | 0,061 ml/min. |
| Viscosité dynamique (vitesse des fibres) | 30,8 cm/s. |
| Vitesse de rotation du mandrin | 1500 tours/min. |
| Nombre de couches | 200 couches |
| Vitesse de révolution | 24641,7 rév/s/s. |

Préalablement à l'enfilage du tuteur sur le mandrin 9, on peut aussi prévoir l'application de quelques couches de fibres de la manière décrite, directement sur le mandrin, et puis seulement l'enfilage du tuteur sur le mandrin, suivi du procédé de préparation décrit précédemment. Dans ce cas, le tuteur présente un recouvrement non seulement sur sa surface de paroi externe, mais aussi sur sa surface de paroi interne.

Après la sortie de l'enceinte, les tuteurs revêtus sont tronçonnés à la longueur souhaitée.

Si le tuteur utilisé est auto-expansible, on prévoira un mandrin 9 ayant un diamètre juste inférieur au diamètre maximal du tuteur et on enfilera celui-ci sur ce dernier et puis on procédera comme décrit précédemment. Il faut toutefois noter que les paramètres du procédé devront être modifiés pour tenir compte de ce que, dans cette forme de réalisation, le tuteur va ensuite être étiré jusqu'à ce qu'il présente son diamètre d. L'angle α peut donc être prévu un peu plus grand, par exemple de 50° ou davantage, éventuellement de presque 90°, pour permettre d'obtenir après étirage, un angle de 30° entre les fibres et une génératrice du tuteur. Pendant l'étirage du tuteur, les fibres se réarrangent entre elles par diminution de l'angle susdit et également grâce à leur visco-élasticité propres, lorsqu'il s'agit de fibres élastiques. Il en résulte qu'il n'y a aucun chiffonnement ou gaufrement du recouvrement autour du tuteur pendant l'étirage.

## Revendications

1. Endoprothèse luminale (1), comprenant
- un élément de tuteur (2) tubulaire pourvu d'une surface de paroi externe et d'une surface de paroi interne et expansible entre un premier diamètre (d) permettant son passage dans une voie d'un corps humain ou animal et un deuxième diamètre (D) supérieur au premier, et
- un recouvrement expansible (5), appliqué sur une des surfaces de paroi de l'élément de tuteur (2), ce recouvrement étant en une matière fibreuse biologiquement inerte, qui est constituée de fibres formant entre elles un échafaudage, et qui présente des ouvertures supérieures à 30µ,
caractérisée en ce que la matière fibreuse est constituée de fibres orientées sous un angle (β) inférieur à 50°, éventuellement inférieur à 30°, par rapport à une génératrice de l'élément de tuteur tubulaire, lorsque ce dernier présente son premier diamètre (d), et sous un angle supérieur à l'angle (β), lorsque l'élément de tuteur présente son deuxième diamètre.

2. Endoprothèse suivant la revendication 1, caractérisée en ce que les ouvertures susdites sont supérieures à 50µ, lorsque l'élément de tuteur (2) présente son deuxième diamètre (D).

3. Endoprothèse luminale suivant l'une des revendications 1 et 2, caractérisée en ce que la matière fibreuse est constituée de fibres élastiques.

4. Endoprothèse luminale suivant l'une quelconque des revendications 1 à 3, caractérisée en ce que la matière fibreuse est constituée de fibres de polycarbonate, de préférence d'un uréthanne polycarbonaté.

5. Endoprothèse luminale suivant l'une quelconque des revendications 1 à 4, caractérisée en ce que la matière fibreuse est constituée de fibres disposées en plusieurs couches superposées.

6. Endoprothèse luminale suivant la revendication 5, caractérisée en ce que le recouvrement comporte au moins 100 couches superposées de matière fibreuse, de préférence entre 100 et 500 couches.

7. Endoprothèse suivant l'une ou l'autre des revendications 5 et 6, caractérisée en ce que chacune des couches superposées susdites présente des fibres orientées de manière à croiser les fibres d'une couche voisine supérieure et celles d'une couche voisine inférieure.

8. Endoprothèse luminale suivant l'une des revendications 1 à 7, caractérisée en ce que la matière fibreuse est une matière tissée ou tricotée ou respectivement un non-tissé.

9. Endoprothèse luminale suivant l'une des revendications 1 à 8, caractérisée en ce que le deuxième diamètre (D) peut être 2 à 4 fois plus grand que le premier diamètre (d).

10. Endoprothèse suivant l'une quelconque des revendications 1 à 9, caractérisée en ce que l'élément de tuteur (2) présente le recouvrement expansible (5) appliqué sur la surface de paroi externe de l'élément de tuteur et/ou sur sa surface de paroi interne.

11. Endoprothèse suivant l'une quelconque des revendications 1 à 10, caractérisée en ce que l'élément de tuteur (2) comporte deux extrémités (6) dont au moins l'une est, sur une zone limitée de la surface de paroi externe, dépourvue de recouvrement expansible.

12. Endoprothèse suivant la revendication 11, caractérisée en ce que la ou les extrémités (6), dépourvues de recouvrement expansible, de l'élément de tuteur font radialement saillie vers l'extérieur, lorsque l'élément de tuteur (2) présente son deuxième diamètre (D), et en ce qu'elles peuvent alors servir de moyens d'accrochage de l'endoprothèse (1) dans ladite voie du corps.

13. Endoprothèse luminale suivant l'une quelconque des revendications 1 à 12, caractérisée en ce que l'élément du tuteur (2) est expansible par l'application d'une force extérieure sur ce dernier.

14. Endoprothèse luminale suivant l'une quelconque des revendications 1 à 12, caractérisée en ce que l'élément de tuteur (2) est auto-expansible.

15. Procédé de préparation d'une endoprothèse luminale suivant l'une quelconque des revendications 1 à 13, caractérisé en ce qu'il comprend une application du recouvrement expansible en une matière fibreuse sur au moins une des surfaces de paroi de l'élément de tuteur présentant son premier diamètre, avec un échafaudage prédéterminé des fibres tel que, lorsque l'élément de tuteur présente son premier diamètre, les fibres sont orientées sous un angle (β) inférieur à 50°, éventuellement inférieur à 30°, par rapport à une génératrice de l'élément de tuteur tubulaire et, lorsque l'élément de tuteur présente son deuxième diamètre, les fibres sont orientées sous un angle supérieur à l'angle (β) et la matière fibreuse présente des ouvertures entre fibres supérieures à 30µ, de préférence supérieures à 50µ.

16. Procédé suivant la revendication 15, caractérisé en ce qu'il comprend un entraînement en rotation autour d'un axe longitudinal de l'élément de tuteur tubulaire présentant son premier diamètre, et l'application par couches successives de fibres de la matière fibreuse sur la surface de paroi externe de l'élément de tuteur en rotation suivant un angle inférieur à 50°, éventuellement inférieur à 30°, par rapport à cet axe longitudinal.

17. Procédé suivant la revendication 16, caractérisé en ce qu'il comprend, préalablement à l'entraînement en rotation susdit de l'élément de tuteur, un entraînement en rotation autour d'un axe longitudinal d'un mandrin présentant un diamètre inférieur audit premier diamètre, une application par couches successives de fibres de la matière fibreuse sur le mandrin en rotation suivant un angle inférieur à 50°, éventuellement à 30°, par rapport à l'axe longitudinal du mandrin, un enfilage de l'élément de tuteur sur ces couches successives de fibres de la matière fibreuse entourant le mandrin, de façon qu'elles soient appliquées sur la surface de paroi interne de l'élément de tuteur, et un enlèvement ultérieur du mandrin.

18. Procédé suivant l'une quelconque des revendications 15 à 17, caractérisé en ce qu'il comprend une introduction à l'intérieur de l'endoprothèse d'un élément gonflable et un gonflement de cet élément gonflable pour faire passer l'élément de tuteur de son premier diamètre à son deuxième diamètre.

19. Procédé de préparation d'une endoprothèse luminale suivant l'une quelconque des revendications 1 à 12 et 14, caractérisé en ce qu'il comprend
- une application du recouvrement expansible en une matière fibreuse sur au moins une des surfaces de paroi de l'élément de tuteur présentant un troisième diamètre égal ou supérieur à son deuxième diamètre, avec un échafaudage prédéterminé des fibres tel que, lorsque l'élément de tuteur présente son deuxième diamètre, la matière fibreuse présente des ouvertures entre fibres supérieures à 30 µm, de préférence supérieures à 50 µm, et
- un étirage de l'élément de tuteur pourvu du recouvrement expansible jusqu'à ce qu'il présente son premier diamètre, position dans laquelle les fibres de la matière fibreuse sont orientées sous un angle (β) inférieur à 50°, éventuellement inférieur à 30°, par rapport à une génératrice de l'élément de tuteur tubulaire, ces fibres étant orientées sous un angle supérieur à l'angle (β) lorsque l'élément de tuteur présente son deuxième diamètre.

20. Procédé suivant la revendication 19, caractérisé en ce qu'il comprend un entraînement en rotation autour d'un axe longitudinal de l'élément de tuteur tubulaire présentant son troisième diamètre, et l'application par couches successives de fibres de la matière fibreuse sur l'élément de tuteur en rotation suivant un angle compris entre 50 et 90° par rapport à cet axe longitudinal.

21. Procédé suivant la revendication 20, caractérisé en ce qu'il comprend, préalablement à l'entraînement en rotation susdit de l'élément de tuteur, un entraînement en rotation autour d'un axe longitudinal d'un mandrin présentant un diamètre inférieur audit troisième diamètre, une application par couches successives de fibres de la matière fibreuse sur le mandrin en rotation suivant un angle compris entre 50 et 90° par rapport à l'axe longitudinal du mandrin, un enfilage de l'élément de tuteur sur ces couches successives de fibres de la matière fibreuse entourant le mandrin, de façon qu'elles soient appliquées sur la surface de paroi interne de l'élément de tuteur, et un enlèvement ultérieur du mandrin.

22. Procédé suivant l'une quelconque des revendications 15 à 21, caractérisé en ce que l'application du recouvrement expansible comporte une superposition de couches de fibres orientées de manière que les fibres d'une couche croisent les fibres d'une couche voisine supérieure et celles d'une couche voisine inférieure.

23. Procédé suivant l'une quelconque des revendications 15 à 22, caractérisé en ce que l'application du recouvrement expansible comporte un thermoscellage entre elles des fibres de la matière fibreuse appliquée.

## Claims

1. A luminal endoprosthesis (1), comprising :
- a tubular support element (2) provided with an external wall surface and an internal wall surface and expandable from a first diameter (d) at which it can be inserted into a human or animal body pathway and a second diameter (D) greater than the first, and
- an expandable coating (5) applied onto one of said external and internal wall surfaces of the support element (2), said coating being made from a biologically inert fibrous material, which is comprised of fibres forming a structure and which has openings greater than 30µ, characterized by the fact that the fibrous material is comprised of fibres oriented at an angle (β) lower than 50°, optionally lower than 30°, with respect to a generating line of the tubular support element, when the latter is at its first diameter (d), and at an angle greater than the angle (β), when the support element is at its second diameter.

2. Luminal endoprosthesis, in accordance with claim 1, characterized by the fact that said openings are greater than 50µ, when the support element (2) is at its second diameter (D).

3. Luminal endoprosthesis in accordance with either claims 1 and 2, characterized by the fact that the fibrous material is made from elastic fibres.

4. Luminal endoprosthesis in accordance with any of claims 1 to 3, characterized by the fact that the fibrous material is made from polycarbonate fibres, preferably polycarbonated urethane.

5. Luminal endoprosthesis in accordance with any of claims 1 to 4, characterized by the fact that the fribrous material is made from fibres arranged in several superimposed layers.

6. Luminal endoprosthesis in accordance with claim 5, characterized by the fact that the coating consists of at least 100 layers of superimposed fibrous material, preferably between 100 and 500 layers.

7. Luminal endoprosthesis in accordance with one or the other of claims 5 to 6, characterized by the fact that the fibres in each of the superimposed layers are oriented in such a manner that they cross the fibres of the upper adjacent layer and those of the lower adjacent layer.

8. Luminal endoprosthesis in accordance with any of claims 1 to 7, characterized by the fact that the fibrous material is a woven or knitted or non-woven material.

9. Luminal endoprosthesis in accordance with one of claims 1 to 8, characterized by the fact that the second diameter (D) can be 2 to 4 times greater than the first diameter (d).

10. Endoprosthesis according to any of claims 1 to 9, characterized by the fact that the support element (2) has said expandable coating (5) applied onto the external wall surface of the support element and/or onto the internal wall surface thereof.

11. Endoprosthesis according to any of claims 1 to 10, characterized by the fact that the support element (2) has two ends (6), at least one of which is without expandable coating on a limited zone of the external wall surface.

12. Endoprosthesis according to claim 11, characterized by the fact that the end(s) (6) of the supporting element, without expandable coating, project(s) radially outward, when the support element (2) is at its second diameter (D), and serve(s) as means for the fixation of the endoprosthesis (1) into the body pathway.

13. Luminal endoprosthesis according to any of claims 1 to 12, characterized by the fact that the support element (2) can be expanded by applying an external force.

14. Luminal endoprosthesis according to any of claims 1 to 12, characterized by the fact that the support element (2) is auto-expandable.

15. Process for manufacturing a luminal endoprosthesis according to any of claims 1 to 13, comprising the step of applying the expandable coating from fibrous material onto at least one wall surface of the support element at its first diameter, with a predetermined fibre structure such that, when the support element is at its first diameter, the fibres are oriented at an angle (β) lower than 50°, optionally lower than 30°, with respect to a generating line of the tubular support element and, when the support element is at its second diameter, the fibres are oriented at an angle greater than the angle (β) and the fibrous material has openings between the fibres which are greater than 30µ, preferably greater than 50µ.

16. Process according to claim 15, which comprises rotating the tubular support element around a longitudinal axis, when it is at its first diameter, and applying successive layers of fibres onto the external wall surface of the rotating support element at an angle of less than 50°, optionally less than 30°, with respect to said longitudinal axis.

17. Process according to claim 16, characterized by the fact that, before the support element is brought into rotation, it comprises rotating a mandrel having a diameter less than said first diameter around a longitudinal axis, applying successive layers of fibres of the fibrous material onto the rotating mandrel at an angle less than 50°, optionally 30°, with respect to the longitudinal axis of the mandrel, placing the support element onto these successive layers of fibres surrounding the mandrel in such a manner that they are applied onto the internal wall surface of the support element, and subsequently removing the mandrel.

18. Prcess according to any of claims 15 to 17, comprising the step of inserting an inflatable element into the endoprosthesis, and of inflating said inflatable element in such a manner that the support element expands from its first diameter to its second diameter.

19. Process for manufacturing a luminal endoprosthesis according to any of claims 1 to 12 and 14, comprising the steps of
- applying the expandable coating from fibrous material onto at least one wall surface of the support element having a third diameter equal to or greater than its second diameter, with a predetermined fibre structure such that, when the support element is at its second diameter, the fibrous material has openings between the fibres which are greater than 30µ, preferably greater than 50µ, and
- stretching the support element provided with the expandable coating until it reaches its first diameter, position wherein the fibres of the fibrous material are oriented at an angle (β) less than 50°, optionally less than 30°, with respect to a generating line of the tubular support element, said fibres being oriented at an angle greater than the angle (β) when the support element is at is second diameter.

20. Process according to claim 19, comprising the step of rotating the tubular support around a longitudinal axis when it is at its third diameter, and applying successive layers of fibres of the fibrous material onto the rotating support element at an angle between 50° and 90° with respect to said longitudinal axis.

21. Process according to claim 20 characterized by the fact that, before the support element is brought into rotation, it comprises rotating a mandrel with a diameter lower than the third diameter around a longitudinal axis, applying successive layers of fibres of the fibrous material onto the rotating mandrel at an angle between 50 and 90° with respect to the longitudinal axis of the mandrel, placing the support element on these successive layers of fibres of the fibrous material surrounding the mandrel, in such a manner that they are applied onto the internal wall surface of the support element, and subsequently removing the mandrel.

22. Process according to any of claims 15 to 21, characterized by the fact that the step of applying an expandable coating comprises a superimposition of layers of fibres oriented in such an manner that the fibres of one layer intersect the fibres of an upper adjacent layer and those of a lower adjacent layer.

23. Process according to any of claims 15 to 22, characterized by the fact that the step of applying an expandable coating comprises thermosealing the fibres of the applied fibrous material.

## Patentansprüche

1. Luminale Endoprothese (1), die aufweist:
- ein röhrenförmiges Stützelement (2), das mit einer äußeren Wandfläche und einer inneren Wandfläche versehen ist und zwischen einem ersten Durchmesser (d), der dessen Durchgang in einen Kanal eines menschlichen oder tierischen Körpers ermöglicht, und einem zweiten Durchmesser (D), der größer ist als der erste, expandierbar ist, und
- eine expandierbare Abdeckung (5), die auf einer der Wandflächen des Stützelements (2) angebracht ist, wobei diese Abdeckung aus einem biologisch inerten, faserigen Material besteht, das aus Fasern gebildet ist, die untereinander ein Gerüst bilden, und die Öffnungen größer als 30 µm aufweist,
dadurch gekennzeichnet, daß das faserige Material aus Fasern gebildet ist, die unter einem Winkel (β) kleiner als 50°, gegebenenfalls kleiner als 30°, ausgerichtet sind, bezüglich einer Generatrix des röhrenförmigen Stützelements, wenn letzteres seinen ersten Durchmesser (d) aufweist, und unter einem Winkel, der größer ist als der Winkel (β), wenn das Stützelement seinen zweiten Durchmesser aufweist.

2. Endoprothese nach Anspruch 1, dadurch gekennzeichnet, daß die oben genannten Öffnungen größer sind als 50 µm, wenn das Stützelement (2) seinen zweiten Durchmesser (D) aufweist.

3. Luminale Endoprothese nach einem der Ansprüche 1 und 2, dadurch gekennzeichnet, daß das faserige Material aus elastischen Fasern besteht.

4. Luminale Endoprothese nach einem der Ansprüche 1 bis 3, dadurch gekennzeichnet, daß das faserige Material aus Polycarbonatfasern, vorzugsweise aus einem Urethanpolycarbonat besteht.

5. Luminale Endoprothese nach einem der Ansprüche 1 bis 4, dadurch gekennzeichnet, daß das faserige Material aus Fasern besteht, die in mehreren übereinanderliegenden Schichten angeordnet sind.

6. Luminale Endoprothese nach Anspruch 5, dadurch gekennzeichnet, daß die Abdeckung wenigstens 100 übereinanderliegende Schichten aus faserigem Material, vorzugsweise zwischen 100 und 500 Schichten aufweist.

7. Endoprothese nach einem der Ansprüche 5 und 6, dadurch gekennzeichnet, daß jede der oben genannten übereinanderliegenden Schichten Fasern aufweist, die so ausgerichtet sind, daß sie die Fasern einer oberen benachbarten Schicht und die einer unteren benachbarten Schicht kreuzen.

8. Luminale Endoprothese nach einem der Ansprüche 1 bis 7, dadurch gekennzeichnet, daß das faserige Material ein gewebtes Material oder Maschenmaterial bzw. ein Vlies-Material ist.

9. Luminale Endoprothese nach einem der Ansprüche 1 bis 8, dadurch gekennzeichnet, daß der zweite Durchmesser (D) 2 bis 4 mal größer sein kann als der erste Durchmesser (d).

10. Endoprothese nach einem der Ansprüche 1 bis 9, dadurch gekennzeichnet, daß das Stützelement (2) die expandierbare Abdeckung (2) aufweist, die auf der äußeren Wandfläche des Stützelements und/oder auf seiner inneren Wandfläche angebracht ist.

11. Endoprothese nach einem der Ansprüche 1 bis 10, dadurch gekennzeichnet, daß das Stützelement (2) zwei Enden (6) aufweist, wobei wenigstens eines, in einem begrenzten Bereich der äußeren Wandfläche, nicht mit der expandierbaren Abdeckung versehen ist.

12. Endoprothese nach Anspruch 11, dadurch gekennzeichnet, daß das oder die nicht mit expandierbarer Abdeckung versehenen Enden (6) des Stützelements radial nach außen vorragen, wenn das Stützelement (2) seinen zweiten Durchmesser (D) aufweist, und daß sie dann als Befestigungsmittel der Endoprothese (1) in dem Kanal des Körpers dienen können.

13. Luminale Endoprothese nach einem der Ansprüche 1 bis 12 dadurch gekennzeichnet, daß das Stützelement (2) durch Anwendung einer äußeren Kraft auf letzteres expandierbar ist.

14. Luminale Endoprothese nach einem der Ansprüche 1 bis 12, dadurch gekennzeichnet, daß das Stützelement (2) autoexpandierbar ist.

15. Verfahren zur Herstellung einer luminalen Endoprothese nach einem der Ansprüche 1 bis 13, dadurch gekennzeichnet, daß es ein Anbringen der expandierbaren Abdeckung aus einem faserigen Material auf wenigstens eine der Wandflächen des seinen ersten Durchmesser aufweisenden Stützelements aufweist, mit einem vorbestimmten Gerüst der Fasern so, daß wenn das Stützelement seinen ersten Durchmesser aufweist, die Fasern unter einem Winkel (β) kleiner als 50°, gegebenenfalls kleiner als 30°, ausgerichtet sind bezüglich einer Generatrix des röhrenförmigen Stützelements, und wenn das Stützelement seinen zweiten Durchmesser aufweist, die Fasern unter einem Winkel ausgerichtet sind, der größer ist als der Winkel (β), und das faserige Material Öffnungen größer als 30 µm, vorzugsweise größer als 50 µm zwischen Fasern aufweist.

16. Verfahren nach Anspruch 15, dadurch gekennzeichnet, daß es ein rotierendes Antreiben um eine Längsachse des röhrenförmigen, seinen ersten Durchmesser aufweisenden Stützelements und das Anbringen durch aufeinanderfolgende Faserschichten des faserigen Materials auf die äußere Wandfläche des sich drehenden Stützelements gemäß einem Winkel kleiner als 50°, gegebenenfalls kleiner als 30°, bezüglich dieser Längsachse beinhaltet.

17. Verfahren nach Anspruch 16, dadurch gekennzeichnet, daß es vor dem oben genannten rotierenden Antreiben des Stützelements ein rotierendes Antreiben um eine Längsachse eines Dorns mit einem Durchmesser, der kleiner ist als der erste Durchmesser, ein Anbringen durch aufeinanderfolgende Faserschichten des faserigen Materials auf den sich drehenden Dorn gemäß einem Winkel kleiner 50°, gegebenenfalls kleiner als 30°, bezüglich der Längsachse des Dorns, ein Einfädeln des Stützelements auf diese den Dorn umgebenden, aufeinanderfolgenden Faserschichten des faserigen Materials, derart, daß sie auf die innere Wandfläche des Stützelements wirken, und ein späteres Abheben des Dorns beinhaltet.

18. Verfahren nach einem der Ansprüche 15 bis 17, dadurch gekennzeichnet, daß es das Einführen ins Innere der Endoprothese eines aufblasbaren Elements und ein Aufblasen dieses aufblasbaren Elements beinhaltet, um das Stützelement von seinem ersten Durchmesser auf seinen zweiten Durchmesser zu bringen.

19. Verfahren zur Herstellung einer luminalen Endoprothese nach einem der Ansprüche 1 bis 12 und 14, dadurch gekennzeichnet, daß es beinhaltet:
- ein Anbringen der expandierbaren Abdeckung aus einem faserigen Material auf wenigstens eine der Wandflächen des Stützelements, das einen dritten Durchmesser aufweist, der gleich oder größer ist als dessen zweiter Durchmesser, mit einem vorbestimmten Gerüst der Fasern, so daß, wenn das Stützelement seinen zweiten Durchmesser aufweist, das faserige Material Öffnungen zwischen Fasern größer als 30 µm, vorzugsweise größer als 50 µm aufweist, und
- ein Auseinanderziehen des mit der expandierbaren Abdeckung versehenen Stützelements bis es seinen ersten Durchmesser aufweist, eine Position, in der die Fasern des faserigen Material ausgerichtet sind unter einem Winkel (β) kleiner als 50°, gegebenenfalls kleiner als 30°, bezüglich einer Generatrix des röhrenförmigen Stützelements, wobei diese Fasern unter einem Winkel ausgerichtet sind, der größer ist als der Winkel (β), wenn das Stützelement seinen zweiten Durchmesser aufweist.

20. Verfahren nach Anspruch 19, dadurch gekennzeichnet, daß es ein rotierendes Antreiben um eine Längsachse des seinen dritten Durchmesser aufweisenden, röhrenförmigen Stützelements und das Anbringen durch aufeinanderfolgende Faserschichten des faserigen Materials auf das sich drehende Stützelement gemäß einem Winkel zwischen 50° und 90° bezüglich dieser Längsachse beinhaltet.

21. Verfahren nach Anspruch 20, dadurch gekennzeichnet, daß es vor dem oben genannten rotierenden Antreiben des Stützelements ein rotierendes Antreiben um eine Längsachse eines Dorns mit einem Durchmesser, der kleiner ist als der dritte Durchmesser, ein Anbringen durch aufeinanderfolgende Faserschichten des faserigen Materials auf den sich drehenden Dorn gemäß einem Winkel zwischen 50° und 90° bezüglich der Längsachse des Dorns, ein Einfädeln des Stützelements auf diese den Dorn umgebenden, aufeinanderfolgenden Faserschichten des faserigen Materials, derart, daß sie auf die innere Wandfläche des Stützelements wirken, und ein späteres Abheben des Dorns beinhaltet.

22. Verfahren nach einem der Ansprüche 15 bis 21, dadurch gekennzeichnet, daß das Anbringen der expandierbaren Abdeckung ein Übereinanderlegen von Faserschichten beinhaltet, die so ausgerichtet sind, daß die Fasern einer Schicht die Fasern einer benachbarten oberen Schicht und die einer benachbarten unteren Schicht kreuzen.

23. Verfahren nach einem der Ansprüche 15 bis 22, dadurch gekennzeichnet, daß das Anbringen der expandierbaren Abdeckung ein Heißsiegeln untereinander der Fasern des verwendeten faserigen Materials beinhaltet.
